# EUROPEAN PATENT SPECIFICATION

(11) **EP 1 304 107 B1**
(45) Date of publication and mention of the grant of the patent: **06.07.2005**
(21) Application number: 02028171.3
(22) Date of filing: 10.11.1998
(51) Int. Cl.: A61K 31/422, A61K 31/444, A61K 31/4439, A61K 31/496, A61K 31/5377, A61P 17/06

(54) **Use of oxazolidinines derivatives for treating psoriasis**
Verwendung von Oxazolidinonderivaten zur Behandlung von Psoriasis
Utilisation de dèrives d'oxazolidinone pour traiter le Psoriasis

(30) Priority: 18.11.1997 US 65689 P; 16.01.1998 US 71297 P; 19.02.1998 US 75247 P
(43) Date of publication of application: 23.04.2003
(62) Divisional of application: 98960157.0
(73) Proprietor: Pharmacia & Upjohn Company LLC, Kalamazoo, MI 49001 (US)
(72) Inventor: Batts, Donald H., Kalamazoo, MI 49008 (US); Ulrich, Roger G., Gurnee, IL 60031 (US)
(74) Representative: Perry, Robert Edward

(56) References cited:
- WO-A-93/09103
- WO-A-93/23384
- WO-A-95/14684
- US-A- 5 688 792

## Description

### BACKGROUND OF THE INVENTION

### 1. Field of the Invention

The present invention is the use of known oxazolidinones for the manufacture of a medicament to treat psoriasis.

### 2. Description of the Related Art

US Patents 5,164,510, 5,231,188, 5,565,571, 5,652,238 and 5,688,792 all disclose various oxazolidinone antibiotics which are well known to those skilled in the art.

Psoriasis is a well known condition, a proliferative disease of the skin of unknown etiology. It is not known, or believed, to have a microbiologic cause.

Problems experienced by those suffering with psoriasis include intense itching and discomfort, unsightly skin blemishes and chronic scratching resulting in skin infections.

At present there are no cures, only methods of dealing with the clinical symptoms experienced by the patients. These methods of treatment include avoiding drying of the skin and irritation of skin, use of topical steroid cremes and ointments, use of crude coal tar (1-5% in an ointment base) applied topically, ultraviolet light therapy, topical vitamin D (calcipitriol), oral methotrexate for severe cases (a drawback to this therapy is that methotrexate causes severe liver damage if not careful), use of etretrinate (a synthetic retinoid) for severe cases (however a drawback to this therapy is that it is associated with severe deformities in fetuses if a female patient is pregnant).

There is no cure for psoriasis, but rather treatments which may induce a remission for a period of time. Since the pathogenesis of psoriasis is unknown, the reason why the various treatments do not fully succeed is not known but it is likely that present treatments are not treating the root cause of psoriasis.

While there are number of pharmaceutical agents available for treating psoriasis, none treat the condition/disease as well as psoriasis sufferers or physicians would like.

### SUMMARY OF INVENTION

Disclosed is the use a of an effective amount of an oxazolidinone selected from the group consisting of:
(S)-N-[[3-[3-fluoro-4-[4-(hydroxyacetyl)-1-piperazinyl]-phenyl]-2-oxo-5-oxazolidinyl]methyl]acetamide,
(S)-N-[[3-[3-fluoro-4-(4-morpholinyl)phenyl]-2-oxo-5-oxazolidinyl]methyl]acetamide,
[4(S)-cis]-(-)-N-[[3-[3-Fluoro-4-(tetrahydro-1-oxido-2H-thiopyran-4-yl)phenyl]-2-oxo-5-oxazolidinyl]methyl]acetamide,
N-((5*S*)-3-(3-fluoro-4-(4-(2-fluoroethyl)-3-oxopiperazin-1-yl)phenyl)-2-oxooxazolidin-5-ylmethyl)acetamide,
(*S*)-N-[[3-[5-(3-pyridyl)thiophen-2-yl]-2-oxo-5-oxazolidinyl]methyl]acetamide and
(*S*)-N-[[3-[5-(4-pyridyl)pyrid-2-yl]-2-oxo-5-oxazolidinyl]methyl]acetamide and pharmaceutically acceptable salts thereof for the manufacture of a medicament for treating psoriasis.

### DETAILED DESCRIPTION OF THE INVENTION

The oxazolidinones of the present invention are known, see EXAMPLES 1 thru 6 (OXAZOLIDINONEs) and pharmaceutically acceptable salts thereof.

Suitable pharmaceutical acceptable salts include the acid addition salts from the both inorganic and organic acids including hydrochloric, hydrobromic, sulfuric, phosphoric, sulfonic acids, methanesulfonic, gluconic, galacturonic, citratic, oxylatic and acetic.

It is preferred that the person being treated for psoriasis with OXAZOLIDINONEs does not have a gram positive bacterial infection at the time of being treated.

In treating psoriasis, the OXAZOLIDINONEs can either be used individually or in combination with each other or in combination with non-OXAZOLIDINONEs.

In treating psoriasis, the OXAZOLIDINONEs are administered orally, parenterally (IV=intravenously), topically or rectally.

When administered orally, the OXAZOLIDINONEs can be administered in tablet, capsule or liquid (suspension, syrup or solution) dosage form. Regardless of the dosage form, an anti-psoriasis effective amount of the OXAZOLIDINONEs is from about 50 mg to about 1,200 mg/day. It is preferred that the OXAZOLIDINONEs be given in two or more divided doses, more preferably in two divided doses.

When administered parenterally, the OXAZOLIDINONEs should be administered IV. The IV infusion should be adjusted such that the flow rate delivers an anti-psoriasis effective amount of from about 50 mg to about 1,200 mg/day. (S)-N-[[3-[3-Fluoro-4-(4-morpholinyl)phenyl]-2-oxo-5-oxazolidinyl]methyl]acetamide is virtually 100% bioavailable. Therefore, the patient will achieve approximately the same blood levels with the same dose regardless of whether it is given orally of parenterally.

When applied topically, the OXAZOLIDINONEs can be applied in many different pharmaceutical dosage forms all well known to those skilled in the art. These include as a solution, cream, ointment, gel, lotion, suspension or emulsion, etc. Regardless of the topical pharmaceutical dosage form selected, the concentration of the OXAZOLIDINONEs should be from 0.01% to 10% (wt/wt). Regardless of the pharmaceutical dosage form, the topical formulation should be applied one to four times daily.

When administered rectally, the OXAZOLIDINONEs should be administered as a suppository which delivers an anti-psoriasis effective amount of from about 50 mg to 1,200 mg/day.

When administered systemically, whether orally, parenterally or rectally the OXAZOLIDINONEs should be administered either continuously or in cyclic courses of 7-28 days every 1 to 12 months. When administered topically, the OXAZOLIDINONEs should be applied either daily for 7 to 28 days every 1 - 12 months.

### DEFINITIONS AND CONVENTIONS

The definitions and explanations below are for the terms as used throughout this entire document including both the specification and the claims.

### DEFINITIONS

Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

When the solubility of a solid in a solvent is used the ratio of the solid to the solvent is weight/volume (wt/v).

When the % active ingredient of a pharmaceutical formulation is set forth, it is the ratio of the active ingredient of the entire pharmaceutical formulation and is expressed as weight/weight (wt/wt).

Alcohol refers to ethyl alcohol.

IV refers to parenteral administration by the intravenous route.

OXAZOLIDINONES refers to the compounds of EXAMPLES 1 thru 6.

### EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative.

### EXAMPLE 1 (S)-N-[[3-[3-Fluoro-4-[4-(hydroxyacetyl)-1-piperazinyl]-phenyl]-2-oxo-5-oxazolidinyl]methyl]acetamide

(S)-N-[[3-[3-Fluoro-4-[4-(hydroxyacetyl)-1-piperazinyl]-phenyl]-2-oxo-5-oxazolidinyl]methyl]acetamide is known, see US Patent 5,652,238, EXAMPLE 1.

### EXAMPLE 2 (S)-N-[[3-[3-Fluoro-4-(4-morpholinyl)phenyl]-2-oxo-5-oxazolidinyl]methyl]acetamide

(S)-N-[[3-[3-Fluoro-4-(4-morpholinyl)phenyl]-2-oxo-5-oxazolidinyl]methyl]acetamide is known, see US Patent 5,688,792, EXAMPLE 5.

### EXAMPLE 3 [4(S)-cis]-(-)-N-[[3-[3-Fluoro-4-(tetrahydro-1-oxido-2H-thiopyran-4-yl)phenyl]-2-oxo-5-oxazolidinyl]methyl]acetamide

A mixture of (S)-(-)-N-[[3-[3-fluoro-4-(3,6-dihydro-2H-thiopyran-4-yl)phenyl]-2-oxo-5-oxazolidinyl]methyl]acetamide S-oxide (International Publication No. WO 97/09328, 4.50 g) and platinum oxide (697 mg) in methanol (164 ml) is shaken on the Parr apparatus under a hydrogen atmosphere at 40 psi for 18 hours. The catalyst is then removed by filtration through Celite, and the filtrate is concentrated under reduced pressure and the residue chromatographed on silica gel (230 - 400 mesh, 350 g), eluting with a gradient of methanol/methylene chloride (3/97 - 7/93). The appropriate fractions (those fractions with an R_{f} = 0.44 by TLC; methanol/chloroform, 10/90) are pooled and concentrated to give the title compound, mp 203 - 204°.

### EXAMPLE 4 N-((5S)-3-(3-Fluoro-4-(4-(2-fluoroethyl)-3-oxopiperazin-1-yl)phenyl)-2-oxooxazolidin-5-ylmethyl)acetamide

N-((5*S*)-3-(3-Fluoro-4-(4-(2-fluoroethyl)-3-oxopiperazin-1-yl)phenyl)-2-oxooxazolidin-5-ylmethyl)acetamide is known, see International Publication WO97/27188 (Example 4).

1-*t*-Butoxycarbonyl-3-oxopiperazine (21.6 g) is dissolved in dry DMF (500 ml) and potassium *t*-butoxide (24.2 g) is added. The mixture is stirred at 20-25° for 30 minutes, then 1-(4-methylphenylsulfonyloxy)-2-fluoroethane (*J*. *Med. Chem.,* 23(9), 985-90 (1980), 25.9 g) is added and stirring continued at the same temperature for 24 hours. The solvent is removed and the residue partitioned between ethyl acetate and water. The organic phase is washed with water and concentrated. The residue is dissolved in isopropanol and diluted with iso-hexane forming a precipitate which is removed by filtration. The mixture is chromatographed (silica; eluting with a gradient increasing in polarity from 0 to 50% isopropanol in iso-hexane) to give 1-*t*-butoxycarbonyl-4-(2-fluoroethyl)-3-oxopiperazine.

1-*t*-Butoxycarbonyl-4-(2-fluoroethyl)-3-oxopiperazine (6.65 g) is dissolved in dichloromethane (500 ml), cooled in an ice-bath and trifluoroacetic acid (150 ml) added. The mixture is stirred at the same temperature for 2 hours. The solvent is removed to give a crude product which is dissolved in the minimum volume of ethyl acetate. Slow addition of ether causes precipitation of 1-(2-fluoroethyl)-2-oxopiperazine as the mono trifluoroacetic acid salt.

1-(2-Fluoroethyl)-2-oxopiperazine trifluoroacetate (6.1 g) is dissolved in acetonitrile (100 ml). N,N-Diisopropylethylamine (13 ml) is added to the mixture, followed by 3,4-difluoronitrobenzene (3.39 g) and the mixture heated to reflux for 18 hours. The solvent is removed and the residue chromatographed (silica; eluting with a gradient increasing in polarity from 0 to 4% methanol in dichloromethane) to give 3-fluoro-4-(4-{2-fluoroethyl}-3-oxopiperazin-1-yl)nitrobenzene.

3-Fluoro-4-(4-{2-fluoroethyl}-3-oxopiperazin-1-yl)nitrobenzene (4.35 g) is dissolved in a mixture of ethyl acetate (250 ml) and DMF (5 ml), and the solution flushed with argon. Palladium (10% on carbon, 200 mg) is added and the mixture hydrogenated under ambient pressure. After gas uptake had ceased, the mixture is filtered through celite and solvent removed. The residue is taken up in ethyl acetate, washed twice with water, dried over magnesium sulfate and the solvent is removed to give 5-amino-2-[4-(2-fluoroethyl)-3-oxopiperazin-1-yl]fluorobenzene which is used without further purification.

5-Amino-2-(4-[2-fluoroethyl]-3-oxopiperazin-1-yl)fluorobenzene (2.6 g) is dissolved in dry dichloromethane (50 ml) under argon. Pyridine (1.03 ml) is added, and the mixture cooled to -20°. Benzyl chloroformate (1.6 ml) is added and the mixture stirred for 10 minutes at -20°, before allowing the temperature to rise to 20-25° over 1.5 hours. The solvents are removed and the residue is dissolved in dichloromethane and washed with sodium bicarbonate solution. After drying over magnesium sulfate and removal of the solvent, the residue is chromatographed (silica, eluting with a gradient increasing in polarity from 0 to 5% methanol in dichloromethane) to give 5-benzyloxycarbonylamino-2-(4-[2-fluoroethyl]-3-oxopiperazin-1-yl)fluorobenzene.

A solution of lithium *t*-butoxide is prepared by addition of *n*-butyllithium (1.6 M in hexane, 2.9 ml) to a stirred solution of t-butanol (0.43 g) in anhydrous THF (10 ml) at -10° under argon. After cooling to -70°, a solution of 5-benzyloxycarbonylamino-2-(4-[2-fluoroethyl]-3-oxopiperazin-1-yl)fluorobenzene (1.5 g) in dry THF (15 ml) is added. After 10 minutes, (R)-glycidylbutyrate (0.67 g) in dry THF (15 ml) is added to the resulting mixture, and stirring continued at -70° for 15 minutes, before allowing the temperature to rise to 20-25° over 16 hours. Methanol (10 ml) is added, followed by saturated sodium bicarbonate solution (20 ml) and water (10 ml). The organic phase is separated and extracted into ethyl acetate (3 x 25 ml), washed with saline and dried over magnesium sulfate. The solvent is removed and the residue purified by chromatography (silica; eluting with a gradient increasing in polarity from 0 to 3% methanol in dichloromethane) to give (5R)-3-(3-fluoro-4-[4-(2-fluoroethyl)-3-oxopiperazin-1-yl]phenyl)-5-hydroxymethyloxazolidin-2-one.

(5R)-3-(3-Fluoro-4-[4-(2-fluoroethyl)-3-oxopiperazin-1-yl]phenyl)-5-hydroxymethyloxazolidin-2-one (0.8 g) is dissolved in pyridine (15 ml) and the mixture cooled to 0°. Triethylamine (0.38 ml) and methanesulfonyl chloride (0.19 ml) are added to the mixture, and stirring continued at 20-25° for 2 hours. The solvent is removed and the residue dissolved in dichloromethane, washed with water, saline, dried over magnesium sulfate and concentrated. The resulting residue is triturated with ether to give (5R)-3-(3-fluoro-4-[4-(2-fluoroethyl)-3-oxopiperazin-1-yl]phenyl)-5-(methanesulfonyloxymethyl)oxazolidin-2-one (0.76 g) which is used without further purification.

(5R)-3-(3-Fluoro-4-[4-(2-fluoroethyl)-3-oxopiperazin-1-yl]-5-(methanesulfonyloxymethyl)oxazolidin-2-one (719 mg) is dissolved in dry DMF (15 ml) and sodium azide (647 mg) is added to the mixture. The mixture is heated at 80° for 6 hrs and then concentrated to dryness. The resulting residue is dissolved in ethyl acetate, washed twice with water, and dried over magnesium sulfate. Removal of the solvent gives (5R)-5-azidomethyl-3-(3-fluoro-4-(4-(2-fluoroethyl)-3-oxopiperazin-1-yl)phenyl)oxazolidin-2-one (413 mg) which is used without further purification.

(5R)-5-Azidomethyl-3-(3-fluoro-4-[4-(2-fluoroethyl)-3-oxopiperazin-1-yl]phenyl)oxazolidin-2-one (360 mg) is dissolved in dry DMF (20 ml) and the mixture purged with argon. Palladium (10% on carbon, 72 mg) is added, followed by acetic anhydride (0.17 ml) and the mixture stirred at 20-25° under hydrogen confined in a balloon for 3 hr. The mixture is filtered through celite, concentrated to dryness and partitioned between ethyl acetate and water. The organic extract is washed with saline, dried over magnesium sulfate and concentrated. The residue is chromatographed (silica gel; eluting with a gradient increasing in polarity from 0 to 2.5% methanol/dichloromethane). The appropriate fractions are pooled and concentrated to give the title compound.

### EXAMPLE 5 (S)-N-[[3-[5-(3-Pyridyl)thiophen-2-yl]-2-oxo-5-oxazolidinyl]methyl]acetamide

(*S*)-N-[[3-[5-(3-Pyridyl)thiophen-2-yl]-2-oxo-5-oxazolidinyl]methyl]acetamide is known, see US Patent 5,698,574 (Example 124).

### EXAMPLE 6 (S)-N-[[3-[5-(4-Pyridyl)pyrid-2-yl]-2-oxo-5-oxazolidinyl]methyl]acetamide hydrochloride

(*S*)-N-[[3-[5-(4-Pyridyl)pyrid-2-yl]-2-oxo-5-oxazolidinyl]methyl]acetamide hydrochloride is prepared following the general procedure of US Patent 5,627,181 EXAMPLES 36 and 52 and making non-critical variations but using a 4-pyridinyl adduct.

### EXAMPLE A 47 Year Old White Male - Psoriasis

A 47 year old white male had a history of psoriasis since age 32. He had chronic psoriatic lesions of both elbows, groin, left leg, right leg, and lower back. The lesions were treated in the past with coal tar and topical steroids but not UV light. He was allergic to the excipients in the steroidal topical preparations.

He was treated with 625 mg of (S)-N-[[3-[3-fluoro-4-(4-morpholinyl)phenyl]-2-oxo-5-oxazolidinyl]methyl]acetamide IV twice daily for five days and then with 625 mg of the same pharmaceutical orally twice daily for nine days. Hence, the patient was given 625 mg of (S)-N-[[3-[3-fluoro-4-(4-morpholinyl)phenyl]-2-oxo-5-oxazolidinyl]methyl]acetamide twice daily for fourteen days and noticed an immediate improvement in his psoriasis as soon as he started taking the medication. He had a complete clearing of the psoriasis from the time he took the medication until three months later when it gradually began to return. After six months it had returned to the pretreatment state. During the time he took the oxazolidinone he did not use any other psoriasis treatment.

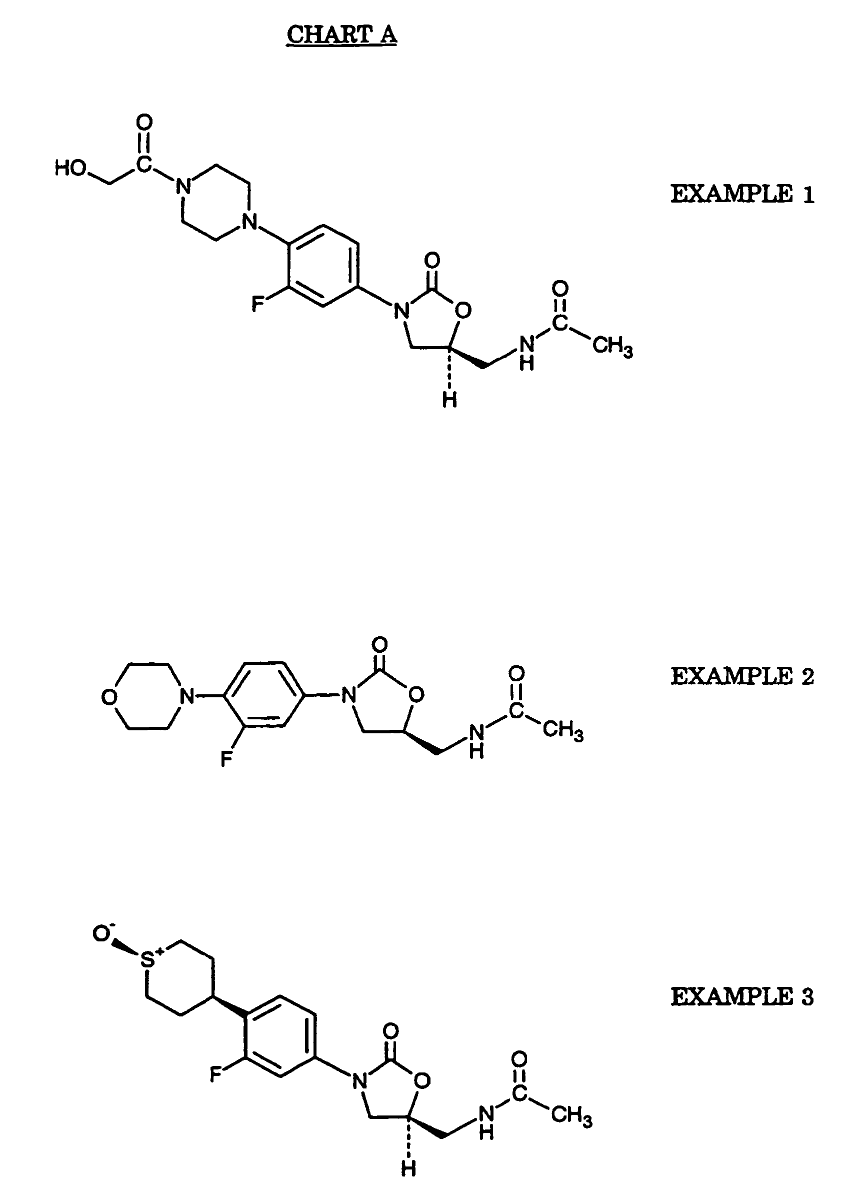

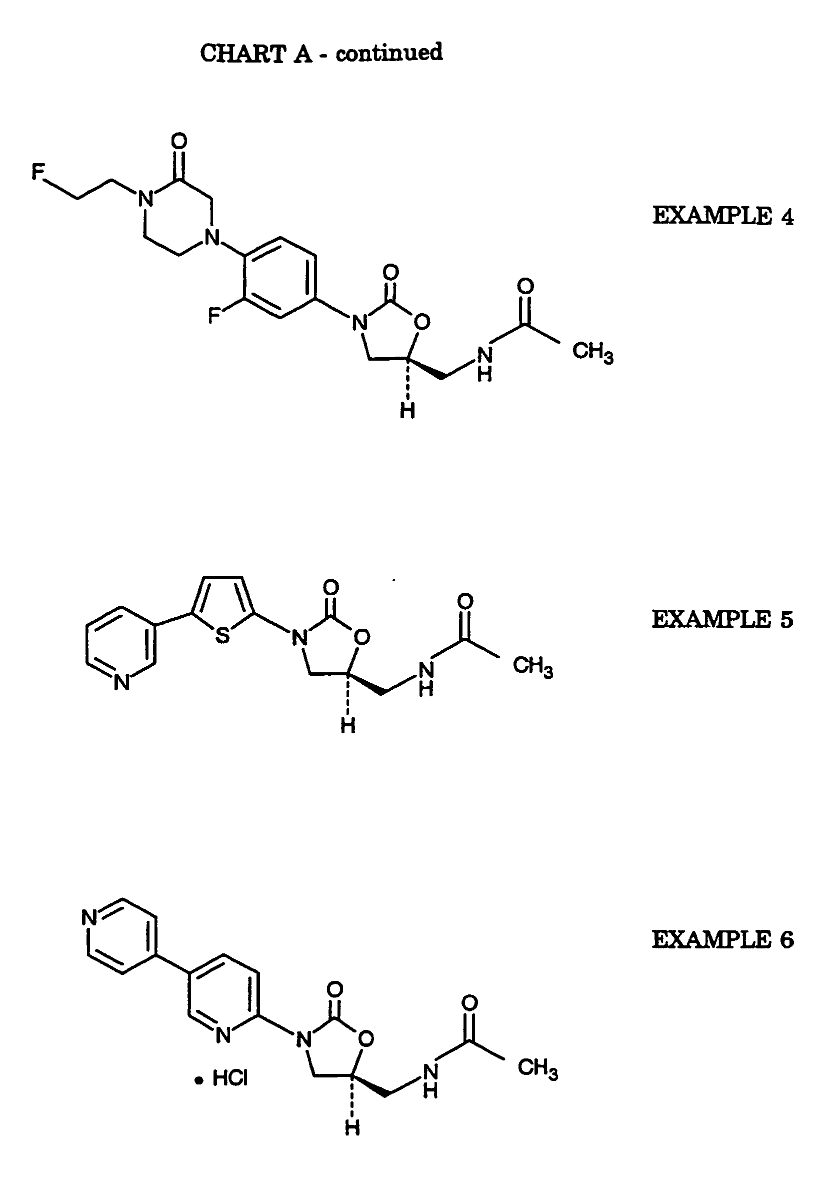

## Claims

1. Use of an oxazolidinone selected from
(*S*)-*N*-[[3-[3-fluoro-4-[4-(hydroxyacetyl)-1-piperazinyl]phenyl]-2-oxo-5-oxazolidinyl]methyl]acetamide,
(*S*)-*N*-[[3-[3-fluoro-4-[4-(morpholinyl)phenyl]-2-oxo-5-oxazolidinyl]methyl]-acetamide,
[4(*S*)-cis]-(-)-*N*-[[3-[3-fluoro-4-(tetrahydro-1-oxido-2H-thiopyran-4-yl)phenyl]-2-oxo-5-oxazolidinyl]methyl]acetamide,
*N*-((5*S*)-3-(3-fluoro-4-(4-(2-fluoroethyl)-3-oxopiperazin-1-yl)phenyl)-2-oxooxazolidin-5-ylmethyl)acetamide,
(*S*)-*N*-[[3-[3-[5-(3-pyridyl)thiophen-2-yl]-2-oxo-5-oxazolidinyl]methyl]acetamide and
(*S*)-*N*-[[3-[3-[5-(4-pyridyl)pyrid-2-yl]-2-oxo-5-oxazolidinyl]methyl]acetamide and
pharmaceutically acceptable salts thereof for the manufacture of a medicament for treating psoriasis.

2. Use according to claim 1, where the medicament is to be administered orally.

3. Use according to claim 2, where the daily dose of the oxazolidinone is from 50 to 1,200 mg/day

4. Use according to claim 1, where the medicament is to be administered topically as a solution, cream, ointment, gel, lotion, suspension or emulsion.

5. Use according to claim 4, where the amount to be administered topically is from 01% to 10%.

6. Use according to claim 1, where the medicament is to be administered intravenously.

7. Use according to claim 6, where the amount to be administered intravenously is from 50 to 1,200 mg/day.

8. Use according to any preceding claim, where the oxazolidinone is (*S*)-*N*-[[3-[3-[fluoro-4-(4-morpholinyl)phenyl]-2-oxo-5-oxazolidinyl]methyl]acetamide.

## Patentansprüche

1. Verwendung eines Oxazolidinons, das aus
(S)-N-[[3-[3-Fluor-4-[4-(hydroxyacetyl)-1-piperazinyl]-phenyl]-2-oxo-5-oxazolidinyl]methyl]acetamid,
(S)-N-[[3-[3-Fluor-4-[4-(morpholinyl)phenyl]-2-oxo-5-oxazolidinyl]methyl]acetamid,
[4(S)-cis]-(-)-N-[[3-[3-Fluor-4-(tetrahydro-1-oxido-2H-thiopyran-4-yl)phenyl]-2-oxo-5-oxazolidinyl]methyl]-acetamid,
N-((5S)-3-(3-Fluor-4-(4-(2-fluorethyl)-3-oxopiperazin-1-yl)phenyl)-2-oxooxazolidin-5-ylmethyl)acetamid,
(S)-N-[[3-[3-[5-(3-Pyridyl)thiophen-2-yl]-2-oxo-5-oxazolidinyl]methyl]acetamid
und
(S)-N-[[3-[3-[5-(4-Pyridyl)pyrid-2-yl]-2-oxo-5-oxazolidinyl]methyl]acetamid
ausgewählt ist, und von pharmazeutisch akzeptablen Salzen derselben zur Herstellung eines Medikaments zur Behandlung von Psoriasis.

2. Verwendung nach Anspruch 1, wobei das Medikament oral zu verabreichen ist.

3. Verwendung nach Anspruch 2, wobei die Tagesdosis des Oxazolidinons 50 bis 1200 mg/Tag beträgt.

4. Verwendung nach Anspruch 1, wobei das Medikament topisch als Lösung, Creme, Salbe, Gel, Lotion, Suspension oder Emulsion zu verabreichen ist.

5. Verwendung nach Anspruch 4, wobei die topisch zu verabreichende Menge 0,01 bis 10 % beträgt.

6. Verwendung nach Anspruch 1, wobei das Medikament intravenös zu verabreichen ist.

7. Verwendung nach Anspruch 6, wobei die intravenös zu verabreichende Menge 50 bis 1200 mg/Tag beträgt.

8. Verwendung nach einem der vorhergehenden Ansprüche, wobei das Oxazolidinon (S)-N-[[3-[3-Fluor-4-[4-(morpholinyl)phenyl]-2-oxo-5-oxazolidinyl]methyl]acetamid ist.

## Revendications

1. Utilisation d'une oxazolidinone choisie entre :
*(S)*-*N*-[[3-[3-fluoro-4-[4-(hydroxyacétyl)-1-pipérazinyl]-phényl]-2-oxo-5-oxazolidinyl]méthyl]acétamide,
*(S)*-*N*-[[3-[3-fluoro-4-[4-(morpholinyl)phényl]-2-oxo-5-oxazolidinyl]méthyl]acétamide,
[4*(S)*-cis]-(-)-*N*-[[3-[3-fluoro-4-(tétrahydro-1-oxydo-2H-thiopyranne-4-yl)phényl]-2-oxo-5-oxazolidinyl]méthyl] acétamide,
*N*-((5*S*)-3-(3-fluoro-4-(4-fluoréthy)-3-oxopipérazine-1-yl)-phényl)-2-oxo-oxazolidine-5-ylméthyl)acétamide,
*(S)*-*N*-[[3-[3-[5-(3-pyridyl)thiophène-2-yl]-2-oxo-5-oxazolidinyl]méthyl]acétamide, et
*(S)*-*N*-[[3-[3-[5-(4-pyridyl)pyride-2-yl]-2-oxo-5-oxazolidinyl]méthyl]acétamide, et
leurs sels pharmaceutiquement acceptables, pour la production d'un médicament destiné au traitement du psoriasis.

2. Utilisation suivant la revendication 1, dans laquelle le médicament est destiné à être administré par voie orale.

3. Utilisation suivant la revendication 2, dans laquelle la dose quotidienne de l'oxazolidinone est comprise entre 50 et 1200 mg/jour.

4. Utilisation suivant la revendication 1, dans laquelle le médicament est destiné à être administré par voie topique sous forme d'une solution, d'une crème, d'une pommade, d'un gel, d'une lotion, d'une suspension ou d'une émulsion.

5. Utilisation suivant la revendication 4, dans laquelle la quantité à administrer par voie topique est comprise entre 0,01 % et 10 %.

6. Utilisation suivant la revendication 1, dans laquelle le médicament est destiné à être administré par voie intraveineuse.

7. Utilisation suivant la revendication 6, dans laquelle la quantité à administrer par voie intraveineuse est comprise entre 50 et 1200 mg/jour.

8. Utilisation suivant l'une quelconque des revendications précédentes, dans laquelle l'oxazolidinone est le *(S)*-*N*-[[3-[3-[fluoro-4-[4-(morpholinyl)phényl]-2-oxo-5-oxazolidinyl]méthyl]acétamide.
